# EUROPEAN PATENT SPECIFICATION

(11) **EP 1 299 107 B1**
(45) Date of publication and mention of the grant of the patent: **15.06.2005**
(21) Application number: 01960330.7
(22) Date of filing: 19.06.2001
(51) Int. Cl.: A61K 31/5415, A61K 9/08, A61P 11/00, A61P 29/00

(54) **HIGHLY CONCENTRATED STABLE MELOXICAM SOLUTIONS**
HOCHKONZENTRIERTE STABILE MELOXICAM-LÖSUNGEN
SOLUTIONS STABLES FORTEMENT CONCENTREES DE MELOXICAM

(30) Priority: 20.06.2000 DE 10030345
(43) Date of publication of application: 09.04.2003
(73) Proprietor: BOEHRINGER INGELHEIM VETMEDICA GMBH, 55216 Ingelheim am Rhein (DE)
(72) Inventor: DANECK, Klaus, 88400 Biberach (DE); FOLGER, Martin, Andreas, 55218 Ingelheim am Rhein (DE); HASSEL, Bernhard, 55437 Ockenheim (DE); HENKE, Stefan, 55239 Gau-Odernheim (DE); KROFF, Hans-Jürgen, 55444 Schoeneberg (DE); KRUSS, Bernd, 88454 Hochdorf (DE); PROX, Axel, 88447 Warthausen (DE)
(86) International application number: PCT/EP2001/006904
(87) International publication number: WO 2001/097813

(56) References cited:
- EP-A- 0 093 999
- EP-A- 0 177 870
- EP-A- 0 945 134
- WO-A-01/37838
- WO-A-99/59634

## Description

The present invention relates to highly concentrated stable meloxicam solutions for oral and parenteral administration, particularly for treating respiratory diseases in large farm animals.

### Background of the invention

Meloxicam (4-hydroxy-2-methyl-N-(5-methyl-2-thiazolyl)-2H-1,2-benzothiazine-3-carboxamide-1,1-dioxide) is an active substance which belongs to the group of NSAID's (non-steroidal-antiinflammatory drugs). Meloxicam and the sodium and meglumine salt thereof (N-methyl-D-glucamine salt) are described in EP-A-0 002 482. EP-A-0 002 482 shows, inter alia, the example of a 0.2% injectable solution of meloxicam consisting of the meglumine salt of the active substance, sodium chloride and water.

EP-A-0 945 134 discloses the pH-dependent solubility characteristics of meloxicam and its salts, i.e. the sodium salt, the ammonium salt and the meglumine salt, in aqueous solution. According to this, meloxicam is an active substance which does not dissolve readily in water. The meloxicam salts, particularly the meglumine salt, exhibit improved solubility as the pH increases between 4 and 10, as shown in Table 1 of EP-0 945 134. However, up till now it has only been possible to produce stable, clear, aqueous solutions with a low concentration of meloxicam. In addition to the *in situ* formation of a meloxicam salt, e.g. meglumine salt, and the addition of solubilisers, these solutions were required to have a pH in the range of maximum possible solubility as well as being reasonably well tolerated and contain a high proportion of organic solvent. Formulation tests with the same or a similar recipe led to cloudiness of the solution, e.g. if the meloxicam concentrations were higher, e.g. 2%.

WO9959634 A1 describes an eye drop solution containing 0.5% meloxicam but makes no reference to possible meloxicam concentrations over 1%. A commercially available 0.5% meloxicam solution is used in small animals such as dogs, heifers and calves to treat respiratory diseases, for example.

It has not hitherto been possible to treat large farm animals with an injectable meloxicam solution. The low concentration of active substance in the injectable solution did not allow an acceptable, well tolerated injection volume on account of the great weight of the animals. Parenteral administration requires that the solution be free from particles. If there are particles in a parenteral drug, there is a risk of vascular damage or embolism. Organic solvents, solubilisers and watersoluble substances can only be used in certain concentrations for reasons of drug tolerance. The problem of the present invention is to produce particle-free highly concentrated meloxicam solutions which are stable over long periods, suitable for treating farm animals up to 750 kg in weight. The solution should be suitable for administration both orally or parenterally.

### Description of the invention

Surprisingly, it has been found that highly concentrated meloxicam solutions which contain, in addition to a meloxicam salt and certain excipients, another excipient selected from among citric acid, lecithin, gluconic acid, tartaric acid, phosphoric acid and EDTA or the salts thereof, may be produced so as to be particle-free and stable over long periods. The stability was achieved with an unexpectedly small amount of organic solubilisers. The formulation was found to be stable even when subjected to the process of final sterilisation.

This results in the solution to the problem according to the invention, as a formulation of a meloxicam solution which contains, in addition to a meloxicam salt, small concentrations of solubiliser, a preservative, a buffer substance for achieving the optimum pH range and another excipient.

The invention relates, as described in claim 1, to aqueous cyclodextrin-free solutions of meloxicam for parenteral or oral administration which contain a pharmacologically acceptable meloxicam salt of an organic or inorganic base in a highly concentrated solution with 11-25 mg/ml of meloxicam together with suitable excipients. Where the solution contains kDTA or one or more of the alkali metal salts thereof. Subclaims 2-14 describe advantageous further features of the invention.

The formulation according to the invention overcomes the problem arising from the prior art of providing an injectable solution of the active substance meloxicam which is also suitable for treating large farm animals, by permitting a high concentration of active substance in a particle free solution which is stable over the long term, having the composition described hereinafter.

The formulation according to the invention may contain, as the meloxicam salt, the meglumine, sodium, potassium or ammonium salt, preferably the meloxicam meglumine salt.

The solubilisers used may be, for example polyethyleneglycols, polyoxyethylene-polyoxypropylene copolymers (e.g.poloxamer 188), glycofurol, arginine, lysine, castor oil, propyleneglycol, solketal, polysorbate, glycerol, sorbitol, mannitol, xylitol, polyvinylpyrrolidone, lecithin, cholesterol, 12-hydroxystearic acid-PEG660-ester, propyleneglycol monostearate, polyoxy-40-hydrogenated castor oil, polyoxyl-10-oleyl-ether, polyoxyl-20-cetostearylether and polyoxyl-40-stearate or a mixture of sorbitol, mannitol and xylitol, preferably polyethyleneglycols, polyoxyethylene-polyoxypropylene copolymers, glycofurol, polyvinylpyrrolidone, lecithin, cholesterol, 12-hydroxystearic acid-PEG660-esters, propyleneglycol monostearate, polyoxy-40-hydrogenated castor oil, polyoxyl-10-oleyl-ether, polyoxyl-20-cetostearylether and polyoxyl-40-stearate. Particularly preferred are polyethyleneglycols, glycofurol and polyoxyethylene-polyoxypropylene-copolymers, but especially polyethyleneglycols (e.g. Macrogol 300) and polyoxyethylene-polyoxypropylene copolymers (e.g. Poloxamer 188). The preservatives used may be, for example, ethanol, benzoic acid and the sodium or potassium salts thereof, sorbic acid and the sodium or potassium salts thereof, chlorobutanol, benzyl alcohol, phenylethanol, methyl, ethyl, propyl or butyl-p-hydroxybenzoates, phenol, m-kresol, p-chloro-m-kresol or benzalkonium chloride. Particularly preferred are ethanol, benzoic acid and the sodium or potassium salt thereof, sorbic acid and the sodium or potassium salts thereof, chlorobutanol, benzylalcohol, phenylethanol and methyl, ethyl, propyl or butyl p-hydroxybenzoates, but preferably ethanol, benzoic acid and the sodium or potassium salts thereof, sorbic acid and the sodium or potassium salts thereof, but especially ethanol.

The buffer system used to achieve a pH of between 8 and 10 may be, for example, glycine, a mixture of glycine and HCl, a mixture of glycine and sodium hydroxide solution, and the sodium and potassium salts thereof, a mixture of potassium hydrogen phthalate and hydrochloric acid, a mixture of potassium hydrogen phthalate and sodium hydroxide solution or a mixture of glutamic acid and glutamate. Glycine, a mixture of glycine and HCl and a mixture of glycine/sodium hydroxide solution, especially glycine, are particularly preferred.

Other suitable excipients are citric acid, lecithin, gluconic acid, tartaric acid, phosphoric acid and EDTA or the alkali metal salts thereof, preferably tartaric acid and EDTA or the alkali metal salts thereof, particularly disodium EDTA.

One embodiment of the invention contains, in addition to the meglumine or sodium salt of the meloxicam, polyethyleneglycols, glycofurol and/or polyoxyethylene-polyoxypropylene copolymers, but particularly polyethyleneglycols (e.g. Macrogol 300) and/or polyoxyethylene-polyoxypropylene copolymers (e.g. Poloxmer 188) as solubiliser, ethanol, benzoic acid and the sodium or potassium salts thereof or sorbic acid and the sodium or potassium salts thereof, but particularly ethanol, as preservative, and glycine, a mixture of glycine/HCl or a mixture of glycine/sodium hydroxide solution, but preferably glycine, as buffer and disodium EDTA as an additional excipient.

The formulation according to the invention may contain meloxicam in a concentration of 11-25 mg/ml, preferably 13-24 mg/ml, preferably 16-23 mg/ml, particularly preferably 18-22 mg/ml especially 20 mg/ml.

The meglumine concentration may be between 12.5 and 16.5 mg/ml, preferably 13-16 mg/ml, preferably 13.5-15.5 mg/ml, more preferably 14-15 mg/ml, especially about 14 mg/ml. The possible sodium, potassium and ammonium concentrations are calculated accordingly.

The concentration of the solubilisers may be in the range from 20-200 mg/ml, preferably 30-150 mg/ml, preferably 40-130 mg/ml, more preferably 50-120 mg/ml, especially 70-100 mg/ml.

The concentration of the preservative ethanol may be in the range from 100-200 mg/ml, preferably 120-180 mg/ml, more preferably about 150 mg/ml.

The concentration of the preservatives benzoic acid and the sodium or potassium salts thereof, sorbic acid and the sodium or potassium salts thereof, chlorobutanol, benzyl alcohol, phenylethanol, phenol, m-kresol and p-chloro-m-kresol may be in the range from 0.5-50 mg/ml, preferably 1-10 mg/ml, more preferably 3-5 mg/ml.

The concentration of the preservatives benzalkonium chloride, phenylmercurynitrate and methyl-, ethyl-, propyl- or butyl-p-hydroxybenozates may be in the range from 0.01-4 mg/ml, preferably 0.02-3 mg/ml, more preferably 0.1-0.5 mg/ml.

The concentration of the buffer substances may be between 4 and 50 mg/ml, preferably between 5 and 20 mg/ml, more preferably between 8 and 10 mg/ml.

The concentration of the other excipients mentioned above, i.e. EDTA, citric acid, lecithin, gluconic acid, tartaric acid and phosphoric acid or the salts thereof may be in the range from 0.2-3 mg/ml, preferably 0.3-2.5 mg/ml, preferably 0.5-2 mg/ml, most preferably 0.6-1.5 mg/ml, and in particular 0.7-1.0 mg/ml.

Meglumine and meloxicam may be used in a molar ratio of between 9:8 and 12:8, preferably in a molar ratio of 11:8, but especially in a molar ratio of 10:8.

In the formulation according to the invention, meloxicam and the other excipient, particularly disodium EDTA, may be present in a weight ratio of between 25:1 and 15:1, preferably between 24:1 and 16:1, preferably between 23:1 and 17:1, more preferably between 22:1 and 18:1, most preferably between 21:1 and 19:1, in particular about 20:1.

The formulation according to the invention may have shelf-life after opening of 28 days or more.

The solution may have a long term shelf-life of 24 months or more.

The shelf-life of the solution in the sealed original packaging may be 1 month or more, in particular between 1 month and 24 months, but at least between 1 month and 18 months, preferably between 1 month and 12 months, more preferably between 1 month and 9 months, most preferably between 1 month and 6 months, particularly between 1 month and 3 months. Details of the stability tests by way of example can be found in Tables 1 and 2 which follow:

### Test of stability after opening

Packing material: 50 ml colourless glass vials, glass type I, ethylenepropylenenorbornene terpolymer rubber stopper (Type: WI 640 grey), aluminium flanged cap.

### Receipe: analogous to Example 1 of the description

4 ml samples were taken from the storage samples three times a day for six days and on the seventh day 4 ml samples were taken four times. Storage was then continued until 28 days had elapsed and samples were taken again.

**Table 1**

| Test No. | Storage conditions [°C/% relative humidity] | Storage time [Days] | Meloxicam content [mg/ml] |
|---|---|---|---|
| 1 | 25°C | 0 | 19.7 |
| | 25°C/60% | 28 | 19.2 |
| 1 | 25°C | 0 | 20 |
| | 25°C/60% | 28 | 19.2 |

In both samples, in addition to the meloxicam content, the parameters investigated, namely appearance (clear yellow solution), pH (8.0-9.7), ethanol content (13.5-15.75), disodium EDTA content (85.0-110.0 mg/100 ml), sterility (according to Pharm. Eur. and USP) and the stability of the packaging material were found to be unchanged.

### Long term stability test in sealed original packaging

Packaging material: 50 ml colourless glass vials, glass type I, ethylenepropylenenorbornene terpolymer rubber stopper (Type: WI 640 grey), aluminium flanged cap.

Recipe: Analogous to Example 1 of the description.

**Table 2**

| Test No. | Storage conditions [°C/% relative humidity] | Storage time [Days] | Meloxicam content [mg/ml] |
|---|---|---|---|
| 1 | 25°C | 0 | 19.7 |
| | 4°C | 6 | 19.9 |
| | 40°C/75% | 6 | 19.5 |
| | 25°C/60% | 18 | 19.3 |
| | 30°C/70% | 18 | 19.4 |
| 2 | 25°C | 0 | 20.0 |
| | 4°C | 6 | 19.9 |
| | 40°C/75% | 6 | 19.7 |
| | 25°C/60% | 18 | 19.4 |
| | 30°C/70% | 18 | 19.5 |
| | 25°C/60% | 24 | 19.5 |
| | 30°C/70% | 24 | 19.5 |

In both samples, in addition to the meloxicam content, the parameters investigated, namely appearance (clear yellow solution), pH (8.0-9.7), ethanol content (13.5-15.75), disodium EDTA content (85.0-110.0 mg/100 ml), sterility (according to Pharm. Eur. and USP) and the stability of the packaging material were found to be unchanged.

The formulation according to the invention should have a pH of between 8 and 10, preferably between 8.5 and 9, more preferably a pH between 8.7 and 8.9, particularly 8.8.

The formulation according to the invention is suitable for treating pain, inflammation, fever, acute mastitis, diarrhoea, lameness, problems with the locomotor apparatus, and respiratory complaints in animals, preferably acute mastitis, diarrhoea, lameness, problems with the locomotor apparatus and respiratory complaints, especially acute mastitis, diarrhoeaand respiratory complaints, most preferably respiratory complaints. The treatment may be given in conjunction with antibiotic therapy.

The formulation according to the invention is suitable for treating animals, preferably farm animals, more particularly large farm animals.

The formulation according to the invention is suitable for treating animals, preferably animals up to 500 kg, particularly large amimals up to 750 kg.

The dosage of the formulation according to the invention should corresponding to 0.2 to 1.0 mg of active substance per kg of bodyweight, preferably 0.4 to 0.8 mg/kg of bodyweight, more preferably 0.5 to 0.7 mg/kg of bodyweight, particularly preferably 0.6 mg/kg of bodyweight.

The formulation according to the invention may be prepared using the methods of preparing aqueous liquid formulations known from the literature. For example, the appropriate excipients may be added to a meloxicam salt solution.

Various commercial materials for aqueous liquid formulations which will allow sealing under inert gas and final sterilisation by autoclaving in the finished container may be used as a packaging material for the formulation according to the invention. Such materials include for example ampoules or glass vials, particularly glass vials, e.g. 50 ml or 100 ml glass vials of glass Type I (according to Pharm. Eur/USP) in conjunction with rubber stoppers made of ethylenepropylenenorbornene terpolymer (Type WI 640 grey) and aluminium caps.

The meloxicam solutions according to the invention will now be illustrated by the Examples which follow.

### EXAMPLES

### Example 1: 2% meloxicam solution

| Recipe: | |
|---|---|
| | g/l |
| Meloxicam | 20.0 |
| Meglumine | 14.0 |
| Macrogel 300*¹ | 150.0 |
| Poloxamer 188*² | 50.0 |
| Ethanol | 150.0 |
| Glycine | 5.0 |
| EDTA-Na | 1.0 |
| 1M HCl | q.s. ad pH 8.8 |
| 1M NaOH | q.s. ad pH 8.8 |
| Water for injections | ad 1000 ml |

| | |
|---|---|
| *¹ obtainable from Brenntag, Plochingen, Germany | |
| *² obtainable from C.H. Erbsloeh, Krefeld, Germany | |

### Method:

20g of meloxicam are dissolved in 500 ml of an aqueous meglumine solution (14 g/500 ml) at 90°C. The other excipients are added one after another to the solution according to the recipe given above. A pH of 8.8 is then achieved using 1M hydrochloric acid and 1M sodium hydroxide solution. Water is added to the solution until a volume of 1 litre is obtained.

### Example 2: 2% meloxicam solution

| Recipe: | |
|---|---|
| | g/l |
| Meloxicam | 20.0 |
| Meglumine | 12.5 |
| PEG 400 | 100.0 |
| Poloxamer | 50.0 |
| Ethanol | 150.0 |
| Glycine | 5.0 |
| EDTA-Na | 1.0 |
| 1M HCl | q.s.adpH8.8 |
| 1M NaOH | q.s. ad pH 8.8 |
| Water for injections | ad 1000 ml |

### Method:

20g of meloxicam are dissolved in 500 ml of an aqueous meglumine solution (12.5 g/500 ml) at 90°C. The other excipients are added one after another to the solution according to the recipe given above. A pH of 8.8 is then achieved using 1M hydrochloric acid or 1M sodium hydroxide solution. Water is added to the solution until a volume of 1 litre is obtained.

### Example 3: 2.5% meloxicam solution

| Recipe: | |
|---|---|
| | g/l |
| Meloxicam | 25.0 |
| Meglumine | 17.5 |
| PEG 300 | 150.0 |
| Poloxamer | 50.0 |
| Ethanol | 150.0 |
| Glycine | 5.0 |
| EDTA-Na | 1.0 |
| 1M HCl | q.s. ad pH 8.8 |
| 1M NaOH | q.s. ad pH 8.8 |
| Water for injections | ad 1000 ml |

### Method:

25g of meloxicam are dissolved in 500 ml of an aqueous meglumine solution (17.5 g/500 ml) at 90°C. The other excipients are added one after another to the solution according to the recipe given above. A pH of 8.8 is then achieved using 1M hydrochloric acid or 1M sodium hydroxide solution. Water is added to the solution until a volume of 1 litre is obtained.

### Example 4: 1.5% meloxicam solution

| Recipe: | |
|---|---|
| | g/l |
| Meloxicam | 15.0 |
| Meglumine | 10.5 |
| PEG 300 | 100.0 |
| Poloxamer | 50.0 |
| Ethanol | 150.0 |
| Glycine | 5.0 |
| EDTA-Na | 1.0 |
| 1M HCl | q.s. ad pH 8.8 |
| 1M NaOH | q.s. ad pH 8.8 |
| Water for injections | ad 1000 ml |

### Method:

15g of meloxicam are dissolved in 500 ml of an aqueous meglumine solution (10.5 g/500 ml) at 90°C. The other excipients are added one after another to the solution according to the recipe given above. A pH of 8.8 is then achieved using 1M hydrochloric acid or 1M sodium hydroxide solution. Water is added to the solution until a volume of 1 litre is obtained.

### Example 5: 2% meloxicam solution

| Recipe: | |
|---|---|
| | g/l |
| Meloxicam | 20.0 |
| Meglumine | 14.0 |
| PEG 300 | 150.0 |
| Poloxamer | 50.0 |
| p-Chloro-m-Kresol | 2.0 |
| Glycine | 5.0 |
| EDTA-Na | 1.0 |
| 1M HCl | q.s. ad pH 8.8 |
| 1M NaOH | q.s. ad pH 8.8 |
| Water for injections | ad 1000 ml |

### Method:

20g of meloxicam are dissolved in 500 ml of an aqueous meglumine solution (14 g/500 ml) at 90°C. The other excipients are added one after another to the solution according to the recipe given above. A pH of 8.8 is then achieved using 1M hydrochloric acid or 1M sodium hydroxide solution. Water is added to the solution until a volume of 1 litre is obtained.

## Claims

1. Aqueous cyclodextrin-free solution of meloxicam for administration by oral or parenteral route, containing a pharmacologically acceptable meloxicam salt of an organic or inorganic base and one or more suitable excipients, **characterised in that** the meloxicam content is from 11 to 25 mg/ml and the solution contains EDTA or one of the alkali metal salts thereof.

2. Aqueous solution according to claim 1 **characterised in that** the meloxicam salt is the sodium or meglumine salt.

3. Aqueous solution according to claim 2 **characterised in that** it contains meglumine and meloxicam in a molar ratio of between 9:8 and 12:8.

4. Aqueous solution according to claim 3 **characterised in that** it contains meglumine and meloxicam in a molar ratio of 10:8.

5. Aqueous solution according to one of claims 1 to 4, **characterised in that** it contains one or more excipients selected from among the buffers and/or preservatives.

6. Aqueous solution according to one of claims 1 to 5, **characterised in that** it contains one or more excipients selected from among the solubilisers.

7. Aqueous solution according to one of claims 1 to 6, **characterised in that** it contains disodium EDTA.

8. Aqueous solution according to one of claims 1 to 7, **characterised in that** it has a shelf-life after opening of 28 days or more at ambient temperature.

9. Aqueous solution according to one of claims 1 to 8, **characterised in that** it has a long term shelf-life of 24 months or more at ambient temperature in its original packaging.

10. Aqueous solution according to one of claims 1 to 9, **characterised in that** it has a pH of between 8.0 and 10.

11. Aqueous solution according to one of claims 1 to 10, containing meloxicam, meglumine, a polyethyleneglycol, a polyoxyethylene-polyoxypropylene copolymer, ethanol, glycine and optionally sodium hydroxide or hydrochloric acid, **characterised in that** it contains disodium EDTA.

12. Aqueous solution according to one of claims 1 to 10, essentially consisting of meloxicam, meglumine, a polyethyleneglycol, a polyoxyethylene-polyoxypropylene copolymer, ethanol, glycine, water suitable for injection and optionally sodium hydroxide or hydrochloric acid, **characterised in that** it contains disodium EDTA.

13. Use of a solution of meloxicam according to one of claims 1 to 12 for preparing a pharmaceutical composition for treating pain, inflammation, fever and respiratory complaints in large farm animals.

14. Use of a solution according to claim 13, which corresponds to a dosage range of from 0.2 to 1.0 mg of active substance/kg of bodyweight.

## Patentansprüche

1. Wässrige, Cyclodextrin-freie Lösung von Meloxicam für die orale oder parenterale Verabreichung, enthaltend ein pharmakologisch annehmbares Meloxicamsalz einer organischen oder anorganischen Base und einen oder mehrere geeignete Exzipienten, **dadurch gekennzeichnet, dass** der Meloxicam-Gehalt 11 bis 25 mg/ml beträgt und die Lösung EDTA oder ein Alkalimetallsalz davon enthält.

2. Wässrige Lösung nach Anspruch 1, **dadurch gekennzeichnet, dass** das Meloxicamsalz das Natrium- oder Megluminsalz ist.

3. Wässrige Lösung nach Anspruch 2, **dadurch gekennzeichnet, dass** sie Meglumin und Meloxicam in einem Molverhältnis zwischen 9:8 und 12:8 enthält.

4. Wässrige Lösung nach Anspruch 3, **dadurch gekennzeichnet, dass** sie Meglumin und Meloxicam in einem Molverhältnis von 10:8 enthält.

5. Wässrige Lösung nach einem der Ansprüche 1 bis 4, **dadurch gekennzeichnet, dass** sie einen oder mehrere Exzipienten, ausgewählt unter Puffern und/oder Konservierungsmitteln, enthält.

6. Wässrige Lösung nach einem der Ansprüche 1 bis 5, **dadurch gekennzeichnet, dass** sie einen oder mehrere Exzipienten, ausgewählt unter Lösungsvermittlern, enthält.

7. Wässrige Lösung nach einem der Ansprüche 1 bis 6, **dadurch gekennzeichnet, dass** sie Dinatrium-EDTA enthält.

8. Wässrige Lösung nach einem der Ansprüche 1 bis 7, **dadurch gekennzeichnet, dass** sie nach dem Öffnen eine Gebrauchsdauer von 28 Tagen oder mehr bei Umgebungstemperatur hat.

9. Wässrige Lösung nach einem der Ansprüche 1 bis 8, **dadurch gekennzeichnet, dass** sie in ihrer Originalverpackung eine Langzeit-Gebrauchsdauer von 24 Monaten oder mehr bei Umgebungstemperatur hat.

10. Wässrige Lösung nach einem der Ansprüche 1 bis 9, **dadurch gekennzeichnet, dass** sie einen pH zwischen 8,0 und 10 hat.

11. Wässrige Lösung nach einem der Ansprüche 1 bis 10, enthaltend Meloxicam, Meglumin, ein Polyethylenglycol, ein Polyoxyethylen-Polyoxypropylen-Copolymer, Ethanol, Glycin und gegebenenfalls Natriumhydroxid oder Salzsäure, **dadurch gekennzeichnet, dass** die Dinatrium-EDTA enthält.

12. Wässrige Lösung nach einem der Ansprüche 1 bis 10, im wesentlichen bestehend aus Meloxicam, Meglumin, einem Polyethylenglycol, einem Polyoxyethylen-Polyoxypropylen-Copolymer, Ethanol, Glycin, Wasser für Injektionszwecke und gegebenenfalls Natriumhydroxid oder Salzsäure, **dadurch gekennzeichnet, dass** sie Dinatrium-EDTA enthält.

13. Verwendung einer Lösung von Meloxicam nach einem der Ansprüche 1 bis 12 für die Herstellung einer pharmazeutischen Zusammensetzung zur Behandlung von Schmerz, Entzündung, Fieber und Atemwegsstörungen bei Großvieh.

14. Verwendung einer Lösung nach Anspruch 13, welche einem Dosierungsbereich von 0,2 bis 1,0 mg Wirkstoff/kg Körpergewicht entspricht.

## Revendications

1. Solution aqueuse de meloxicam sans cyclodextrine pour l'administration par voie orale ou parentérale contenant un sel pharmacologiquement acceptable de meloxicam et d'une base organique ou inorganique et un ou plusieurs excipients appropriés, **caractérisée en ce que** la teneur en meloxicam est de 11 à 25 mg/ml et la solution contient de l'EDTA ou l'un de ses sels de métaux alcalins.

2. Solution aqueuse selon la revendication 1 **caractérisée en ce que** le sel de meloxicam est le sel de sodium ou de méglumine.

3. Solution aqueuse selon la revendication 2 **caractérisée en ce qu'**elle contient de la méglumine et du meloxicam dans un rapport molaire entre 9 : 8 et 12:8.

4. Solution aqueuse selon la revendication 3 **caractérisée en ce qu'**elle contient de la méglumine et du meloxicam dans un rapport molaire de 10 : 8.

5. Solution aqueuse selon l'une des revendications 1 à 4 **caractérisée en ce qu'**elle contient un ou plusieurs excipients choisis parmi les tampons et/ou les conservateurs.

6. Solution aqueuse selon l'une des revendications 1 à 5 **caractérisée en ce qu'**elle contient un ou plusieurs excipients choisis parmi les solubilisants.

7. Solution aqueuse selon l'une des revendications 1 à 6 **caractérisée en ce qu'**elle contient de l'EDTA de disodium.

8. Solution aqueuse selon l'une des revendications 1 à 7 **caractérisée en ce qu'**elle a une durée de conservation après l'ouverture de 28 jours ou plus à la température ambiante.

9. Solution aqueuse selon l'une des revendications 1 à 8 **caractérisée en ce qu'**elle a une durée de conservation à long terme de 24 mois ou plus à la température ambiante dans son emballage d'origine.

10. Solution aqueuse selon l'une des revendications 1 à 9 **caractérisée en ce qu'**elle a un pH entre 8,0 et 10.

11. Solution aqueuse selon l'une des revendications 1 à 10 contenant du meloxicam, de la méglumine, un polyéthylèneglycol, un copolymère polyoxyéthylène-polyoxypropylène, de l'éthanol, de la glycine et éventuellement de l'hydroxyde de sodium ou de l'acide chlorhydrique, **caractérisée en ce qu'**elle contient de l'EDTA de disodium.

12. Solution aqueuse selon l'une des revendications 1 à 10 consistant essentiellement en meloxicam, méglumine, un polyéthylèneglycol, un copolymère polyoxyéthylène-polyoxypropylène, de l'éthanol, de la glycine, de l'eau convenant pour l'injection et éventuellement de l'hydroxyde de sodium ou de l'acide chlorhydrique, **caractérisée en ce qu'**elle contient de l'EDTA de disodium.

13. Utilisation d'une solution de meloxicam selon l'une des revendications 1 à 12 pour préparer une composition pharmaceutique pour traiter la douleur, une inflammation, la fièvre et les symptômes respiratoires dans les grands animaux de ferme.

14. Utilisation d'une solution selon la revendication 13 qui correspond à une plage posologique de 0,2 à 1,0 mg de substance active/kg de poids corporel.
